# EUROPÄISCHE PATENTSCHRIFT

(11) **EP 0 585 828 B1**
(45) Veröffentlichungstag und Bekanntmachung des Hinweises auf die Patenterteilung: **02.11.1995**
(21) Anmeldenummer: 93113716.0
(22) Anmeldetag: 27.08.1993
(51) Int. Cl.: C07C 403/08

(54) **Verfahren zur Herstellung eines Gemisches von 6-(2,6,6 Trimethyl-cyclohex-1-en-1-yl)-4-methyl-hex-3-en-1-ol und -4-en-1-ol**
Preparation of 6-(2,6,6-trimethyl-cyclohex-1-en-tyl)-4-methyl-hex-3-enol and -4-enol
Procédé pour la préparation de 6-(2,6,6-triméthyl-cyclohex-1-en-1-yl)-4-méthyl-hex-3-énol et -4-énol

(30) Priorität: 03.09.1992 CH 2770/92
(43) Veröffentlichungstag der Anmeldung: 09.03.1994
(73) Patentinhaber: GIVAUDAN-ROURE (INTERNATIONAL) S.A., CH-1214 Vernier (CH)
(72) Erfinder: Helmlinger, Daniel, CH-8600 Dübendorf (CH)
(74) Vertreter: Urech, Peter, Dr.

(56) Entgegenhaltungen:
- EP-A- 0 155 591
- EP-A- 0 403 945
- M FIESER ET AL 'Reagents for Organic Synthesis' 1972 , WILEY-INTERSCIENCE , NEW YORK,US * Seite 260 - Seite 261 *
- SYNTHESIS Nr. 4 , 1977 , STUTTGART DE Seiten 246 - 247 J G DE FRIES ET AL 'Sodium dithiomite as a reductant for aldehydes'

## Beschreibung

Die vorliegende Erfindung betrifft ein neues Verfahren zur Herstellung eines Gemisches von 6-(2',6',6'-Trimethyl-cyclohex-1'-en-1'-yl)-4-methyl-hex-3-en-1-ol und -4-en-1-ol (I), welches als Zwischenprodukt für die Herstellung der u.a. unter den Handelsnamen AMBROX DL ® und SYNAMBRAN ® bekannten Riechstoffe geeignet ist. Diese Riechstoffe bestehen im wesentlichen jeweils aus einem Gemisch von (±)-[3aα,5aβ,9aα,9bβ]-Dodecahydro-3a,6,6,9a-tetramethylnaphtho[2,1-b]furan und (±)-[3aα,5aβ,9aα,9bα]-Dodecahydro-3a,6,6,9a-tetramethylnaphtho[2,1-b]furan und sind geeignete Ersatzprodukte für das teure optisch aktive (-)-[3aR-(3aα,5aβ,9aα,9bβ)]-Dodecahydro-3a,6,6,9a-tetramethylnaphtho[2,1-b]furan, das auf dem Markt unter verschiedenen Handelsnamen käuflich ist, und zwar AMBROX ® (Firmenich), AMBROXAN ® (Henkel), AMBERLYN ® (Quest), SYLVAMBER ® (BASF) sowie AMBROXID ® (Haarman & Reimer).

Ferner betrifft die vorliegende Erfindung ein Verfahren zur Herstellung des oben erwähnten Gemisches von [3aα,5aβ,9aα,9bβ]-Dodecahydro-3a,6,6,9a-tetramethylnaphtho[2,1-b]furan und [3aα,5aβ,9aα,9bα]-Dodecahydro-3a,6,6,9a-tetramethylnaphtho[2,1-b]furan.

Es sind bereits einige Verfahren zur Herstellung von AMBROX ® bekannt, welche von Sclareolid (oder Norambreinolid), d.h. [3aR-(3aα,5aβ,9aα,9bβ)]-Decahydro-3a,6,6,9a-tetramethylnaphtho[2,1-b]furan-2(1H)on, ausgehen. Sclareolid selber wird durch Oxidation, beispielsweise unter Verwendung von Chromsäure, von Sclareol (muskatellem Salbeiöl) hergestellt, und Sclareol seinerseits wird aus dem Naturstoff Salvia sclarea durch Extrahieren gewonnen. Nun ist aber die auf dem Markt erhältliche Menge Sclareol beschränkt, und je nach Ernte von Salvia sclarea kann der Preis beträchtlich schwanken. Einen weiteren Nachteil dieser Verfahren stellt die vom Umweltschutz her problematische Oxidationsstufe von Sclareol zu Sclareolid mit Chromsäure dar.

Weiter bisher bekannte Verfahren zur Herstellung von Dodecahydro-3a,6,6,9a-tetramethylnaphtho[2,1-b]furan verlaufen in der Regel ausgehend von dem in der chemischen Industrie häufig verwendeten und kommerziell erhältlichen "Schlüsselzwischenprodukt" β-Jonon [siehe beispielsweise Japanische Patentpublikation (Kokai) Nr. 81164/1985, Agric. Biol. Chem. 50(6), 1475-1480(1986), Chem.Lett.1981, 757-760, Chem.Lett.1983, 729-732, Europäische Patentpublikation Nr. 165.458, Helv.Chim.Acta 72, 996-1000 sowie Europäische Patentpublikation Nr. 170.955] oder Nerolidol [siehe Deutsche Offenlegungsschrift 3.240.054] und sind deswegen technisch nicht zufriedenstellend, weil viele (mindestens sechs) Reaktionsstufen benötigt werden.

Es bestand somit ein Bedürfnis nach einem technisch realisierbaren Verfahren zur Herstellung des Dodecahydro-3a,6,6,9a-tetramethylnaphtho[2,1-b]furans, das nicht über Sclareol erfolgt und demzufolge nicht von der Verfügbarkeit von Sclareol abhängt, sondern eher aus einem im grossen Massstab hergestellten und deshalb leicht zugänglichen Zwischenprodukt ausgeht und zudem wirtschaftlicher, effizienter und umweltfreundlicher als die bisher verwendeten Verfahren ist. Dies ist nunmehr mittels des erfindungsgemässen Verfahrens möglich, da dieses gerade von dem im grossen Massstab hergestellten und als wichtiges Zwischenprodukt in der technischen Synthese von β-Carotin verwendeten E,E-6-(2',6',6'-Trimethyl-cyclohex-1'-en-1'-yl)-4-methyl-hexa-2,4-dien-1-al, d.h. der Verbindung der Formel 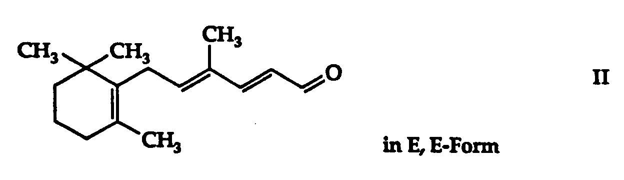
(siehe Carotenoids, Ed.Otto Isler, Birkhäuser Verlag Basel, 1971, Seiten 362-365), ausgeht und (zu den erwünschten obenerwähnten Riechstoffgemischen) nur zwei- oder dreistufig ist.

Bei dem erfindungsgemässen Verfahren handelt es sich um ein Verfahren zur Herstellung eines Gemisches von 6-(2',6',6'-Trimethyl-cyclohex-1'-en-1'-yl)-4-methyl-hex-3-en-1-ol und -4-en-1-ol, d.h. von Verbindungen der Formel 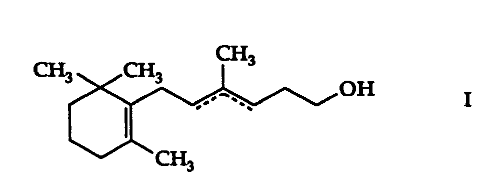
worin jeweils eine der Bindungen 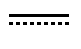 eine einfache Bindung und die andere eine Doppelbindung bezeichnet,
bzw. eines Gemisches von [3aα,5aβ,9aα,9bβ]-Dodecahydro-3a,6,6,9a-tetramethylnaphtho [2,1-b]furan und [3aα,5aβ,9aα,9bα]-Dodecahydro-3a,6,6,9a-tetramethylnaphtho[2,1-b]furan,
das dadurch gekennzeichnet ist, dass man E,E-6-(2',6',6'-Trimethyl-cyclohex-1'-en-1'-yl)-4-methyl-hexa-2,4-dien-1-al entweder mit (a) Lithiumaluminiumhydrid oder mit (b) Natriumdithionit in Gegenwart von Natriumbicarbonat in wässriger Lösung sowie eines Phasentransferkatalysators oder mit (c) Natriumborhydrid oder mit (d) Natrium-bis (2-methoxyäthoxy)aluminiumdihydrid reduziert, und im Falle der Verwendung des Reduktionsmittels (c) oder (d) und gegebenenfalls im Falle der Verwendung des Reduktionsmittels (a) das als Zwischenprodukt entstandene E,E-6-(2',6',6'-Trimethyl-cyclohex-1'-en-1'-yl)-4-methyl-hexa-2,4-dien-1-ol, d.h. die Verbindung der Formel 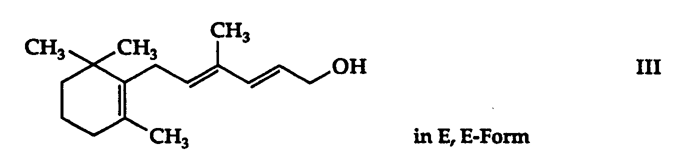
unter Verwendung eines Reduktionsmittels (a) oder (b) zum gewünschten 6-(2',6',6'-Trimethyl-cyclohex-1'-en-1'-yl)-4-methylhex-3-en-1-ol und -4-en-1-ol weiter reduziert,
und dass man gewünschtenfalls die so erhaltenen Verbindungen der Formel I in bekannter Weise in ein Gemisch von [3aα,5aβ,9aα,9bβ]-Dodecahydro-3a,6,6,9a-tetramethylnaphtho[2,1-b]furan und [3aα,5aβ,9aα,9bα]-Dodecahydro-3a,6,6,9a-tetramethylnaphtho [2,1-b]furan überführt.

Im obigen und im folgenden wird die in der Chemie, insbesondere der Carotinoid-Chemie, übliche abgekürzte Darstellung der Strukturformeln benutzt : aliphatische Ketten und Cyclohexenringe werden durch einfache Striche wiedergegeben.

Durch das Vorliegen von aliphatischen C=C-Doppelbindungen in gewissen oben und unten erwähnten Verbindungen kann geometrische Isomerie auftreten, d.h. diese Verbindungen liegen in der cis-(Z-) oder der trans-(E-)Form vor, wobei in den Polyenen jede C=C-Doppelbindung unabhängig in der einen oder der anderen Form vorliegen kann. Zudem hat das mögliche Vorhandensein eines asymmetrischen Kohlenstoffatoms zur Folge, dass die Verbindungen in optisch isomeren Formen (+ oder -) auftreten können. Wenn nicht entsprechend dargestellt, sollen die diesbezüglichen Formeln und Namen die möglichen isomeren Formen sowie Isomerengemische umfassen.

Wie aus der obigen Definition der erfindungsgemässen Erfindung ersichtlich, besteht die jeweilige erste Stufe darin, E,E-6-(2',6',6'-Trimethyl-cyclohex-1'-en-1'-yl)-4-methyl-hexa-2,4-dien-1-al einer Reduktion zu unterwerfen. Je nach verwendetem Reduktionsmittel (a), (b), (c) oder (d) - und im Falle des Reduktionsmittels Lithiumaluminiumhydrid sowie den sonstigen Reaktionsbedingungen - läuft diese Reduktion verschieden ab. Bei der Verwendung von Lithiumaluminiumhydrid (a) wird vor allem je nach Reaktionstemperatur entweder das Zwischenprodukt E,E-6-(2',6',6'-Trimethylcyclohex-1'-en-1'-yl)-4-methyl-hexa-2,4-dien-1-ol (III; bei eher tieferen Temperaturen) bzw. unmittelbar das 6-(2',6',6'-Trimethyl-cyclohex-1'-en-1'-yl)-4-methyl-hex-3-en-1-ol und -4-en-1-ol (I; bei eher höheren Temperaturen) produziert. Hingegen wird bei Verwendung von Natriumdithionit, z.B. Red-Al ® , Vitride ®, (b) immer unmittelbar die Verbindung I, und bei Verwendung von Natriumborhydrid (c) oder Natrium-bis(2-methoxyäthoxy)aluminiumdihydrid, z.B. auch in der Form von N-Aldehydaddukten, z.B. dem Na-Salz der Hydroxymethansulfinsäure (Rongalit ® ), (d) immer unmittelbar die Verbindung III, produziert. In denjenigen Fällen, in denen die Verbindung III das Reduktionsprodukt ist, wird diese anschliessend unter Verwendung des Reduktionsmittels (a) (bei eher höheren Temperaturen) oder (b) in die erwünschten Verbindungen I übergeführt. Die Reaktionsabläufe können wie folgt schematisch dargestellt werden: 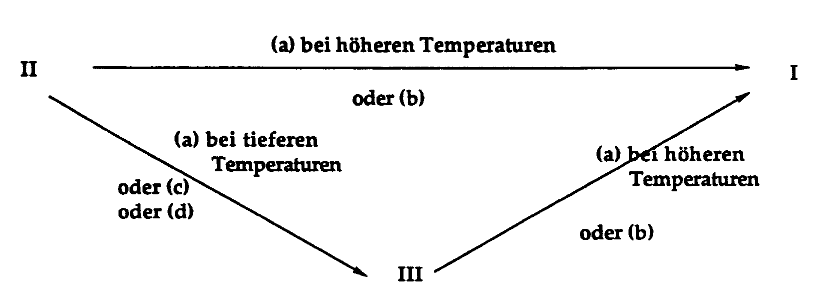
, wobei die jeweiligen zweckmässigen Reaktionsbedingungen nachfolgend erläutert werden.

Im Falle der unmittelbaren Ueberführung der Verbindung der Formel II in die Verbindungen der Formel I mittels Lithiumaluminiumhydrid kann diese zweckmässigerweise durchgeführt werden, indem man die Verbindung der Formel II in Gegenwart des genannten Reduktionsmittels in einem ätherischen Lösungsmittel bei Temperaturen zwischen 65°C und 165°C erhitzt. Geeignete ätherische Lösungsmittel sind diejenigen, die einen Siedepunkt ab etwa 35°C aufweisen. Es kommen in Frage insbesondere aliphatische Aether, z.B.Dimethoxyäthan und Diäthylenglykoldimethyläther, und cyclische Aether, z.B. Tetrahydrofuran, Tetrahydropyran und Dioxan. Vorzugsweise erfolgt die Reduktion bei der Rückflusstemperatur des jeweiligen Reaktionsgemisches. Das Reduktionsmittel wird zweckmässigerweise in leichtem molarem Ueberschuss, d.h. bis zu ca. zehnprozentigem molaren Ueberschuss, eingesetzt. Die sicherste Arbeitsweise besteht darin, dass man eine Lösung der Verbindung der Formel II im ätherischen Lösungsmittel zu einer Lösung des Lithiumaluminiumhydrids in demgleichen Lösungsmittel langsam zutropft, und zwar bei Raumtemperatur. Die Reaktion ist leicht exotherm. Anschliessend wird das Reaktionsgemisch langsam auf die Rückflusstemperatur erhitzt und bis zur Beendigung der Reaktion, die normalerweise mehrere Stunden dauert, am Rückfluss gehalten. Zweckmässigerweise erfolgen das Zutropfen sowie das Erhitzen unter stetigem Rühren des Gemisches.

Falls die Ueberführung der Verbindung der Formel II unter Verwendung von Lithiumaluminiumhydrid in das Zwischenprodukt der Formel III in Kauf genommen wird, so erfolgt die Reduktion eher in einem tiefer siedenden Lösungsmittel bei entsprechend tieferen Temperaturen. In diesem Fall verwendet man zweckmässigerweise einen relativ niedrig siedenden aliphatischen Aether, wie beispielsweise Diäthyläther, und Reaktionstemperaturen von Raumtemperatur bis 40°C. Vorzugsweise führt man die Reduktion bei der Rückflusstemperatur des jeweiligen Reaktionsgemisches durch. Ansonsten benützt man zweckmässigerweise die Arbeitsweise, die oben im Zusammenhang mit der unmittelbaren Ueberführung der Verbindung der Formel II in die Verbindungen der Formel I unter Verwendung von Lithiumaluminiumhydrid beschrieben ist.

Eine unmittelbare Ueberführung der Verbindung der Formel II in die Verbindungen der Formel I wird auch unter Verwendung von Natriumdithionit in Gegenwart von Natriumbicarbonat in wässriger Lösung sowie eines Phasentransferkatalysators erreicht. In diesem Fall kann zusätzlich zum Wasser als Lösungsmittel auch noch ein inertes organisches Lösungsmittel verwendet werden, insbesondere ein alicyclischer Aether, z.B. Tetrahydrofuran oder Dioxan, ein aromatischer Kohlenwasserstoff, z.B. Benzol oder Toluol; oder ein niedriger aliphatischer Ester, z.B. Aethylacetat. Die Menge eingesetzten Natriumdithionits beträgt zweckmässigerweise 1 bis 4 Aequivalente bezogen auf die Menge der Verbindung der Formel II. Man verwendet als Phasentransferkatalysator insbesondere ein quaternäres Ammoniumsalz, z.B. Triäthylbenzylammoniumchlorid, Tetrabutylammoniumhydrogensulphat oder -bromid, Tributylbenzylammoniumchlorid, Tricaprylmethylammoniumchlorid, Trimethyl oder Tributylhexadecylammoniumchlorid oder Trioctylmethylammoniumchlorid, oder ein Phosphoniumsalz, z.B. Hexadecyltributylphosphoniumchlorid, und zwar zweckmässigerweise in einer Menge, die von 3 bis 25 Prozent der molaren Menge des Ausgangsmaterials der Formel II entspricht. Von dem ebenfalls im Reaktionssystem vorliegenden Natriumbicarbonat werden zweckmässigerweise 2 bis 5 Aequivalente eingesetzt, wiederum bezogen auf die Menge der Verbindung der Formel II. Man führt die Reduktion zweckmassigerweise bei der Rückflusstemperatur des Reaktionsgemisches durch.

Bei der Verwendung von Natriumborhydrid als Reduktionsmittel erfolgt die Umsetzung, die zur Verbindung der Formel III führt, zweckmässigerweise in einem Alkohol oder wässrigen Alkohol als Lösungsmittel, wobei der Alkohol insbesondere ein niedriger aliphatischer ist, wie beispielsweise Aethanol, Propanol, Isopropanol, n-Butanol oder tert.Butanol. Zudem wird die Umsetzung zweckmässigerweise bei der Rückflusstemperatur des Reaktionsgemisches durchgeführt. Die Menge eingesetzten Natriumborhydrids beträgt zweckmässigerweise von 0,25 bis 1 Aequivalent, bezogen auf die Menge des Ausgangsmaterials der Formel II.

Auch bei der Verwendung von Natrium-bis(2-methoxyäthoxy)aluminiumdihydrid als Reduktionsmittel erreicht man eine Ueberführung der Verbindung der Formel II in die Verbindung der Formel III. In diesem Fall führt man die Reduktion zweckmässigerweise in einem organischen Lösungsmittel, insbesondere einem aromatischen Kohlenwasserstoff, z.B. Benzol oder Toluol, oder einem cyclischen Aether, z.B. Tetrahydrofuran oder Dioxan, bei der Rückflusstemperatur des Reaktionsgemisches durch. Die Menge eingesetzten Reduktionsmittels beträgt zweckmässigerweise von 0,5 bis 1 Aequivalent bezogen auf die Menge des Ausgangsmaterials.

In den drei Fällen, in denen das Zwischenprodukt der Formel III hergestellt wird, wird dieses erfindungsgemäss weiter in die Verbindung der Formel I übergeführt, und zwar unter Verwendung von Lithiumaluminiumhydrid (bei höheren Temperaturen, insbesondere zwischen 65°C und 165°C) oder Natriumdithionit als Reduktionsmittel. Die diesbezüglichen Reaktionsbedingungen entsprechen im allgemeinen denjenigen, die oben im Zusammenhang mit der unmittelbaren Ueberführung der Verbindung der Formel II in die Verbindungen der Formel I unter Verwendung von Natriumaluminiumhydrid bzw. Natriumdithionit beschrieben sind.

Die jeweilige Aufarbeitung und Isolierung des Reduktionsproduktes der Formel III oder I kann nach an sich bekannten Methoden erfolgen.

Sofern keine gezielte Synthese zur Isolierung reiner Isomerer durchgeführt wird, kann das Produkt als Gemisch zweier oder mehrerer Isomerer anfallen. Dies geschieht ohnehin, wenn das Ausgangsmaterial der Formel II als Isomerengemisch vorliegt. Darüber hinaus fallt das Reduktionsprodukt normalerweise als Gemisch von E- und Z-6-(2',6',6'-Trimethyl-cyclohex-1'-en-1'-yl)-4-methyl-hex-3-en-1-ol sowie E- und/oder Z-6(2',6',6'-Trimethyl-cyclohex-1'-en-1'-yl)-4-methyl-hex-4-en-1-ol an, wobei in der Regel der Hauptteil des Produktes der Formel I aus einem Gemisch von E- und Z-6-(2',6',6'-Trimethyl-cyclohex-1'-en-1'-yl)-4-methyl-hex-3-en-1-ol besteht.

Falls gewünscht können die so hergestellten Isomeren nach an sich bekannten Methoden aufgetrennt werden, wie fraktionierte Kristallisation oder Säulenchromatographie. In der Praxis ist dies jedoch üblicherweise nicht nötig, da das Reduktionsprodukt in einem weiteren Reaktionsschritt in den erwünschten Riechstoff übergeführt und die Auftrennung - falls erwünscht - nach dieser Umwandlung durchgeführt werden kann.

Die Ueberführung der erhaltenen Verbindungen der Formel I in das Gemisch von [3aα,5aβ,9aα,9bβ]-Dodecahydro-3a,6,6,9a-tetramethylnaphtho[2,1-b]furan und [3aα,5aβ,9aα,9bα]-Dodecahydro-3a,6,6,9a-tetramethylnaphtho[2,1-b]furan kann in an sich bekannter Weise erfolgen, insbesondere durch mit Säure katalysierte Cyclisierung des Reduktionsproduktes bei niedrigen Temperaturen in einem inerten organischen Lösungsmittel. Geeignete Reaktionsbedingungen sind beispielsweise in der Japanischen Patentpublikation (Kokai) Nr. 258.773/1990 (unter Verwendung von ca. 1 bis 5 Mol Chlorsulfonsäure als Säurekatalysator pro 1 Mol Ausgangsmaterial und eines Nitroalkans, aliphatischen Halogenkohlenwasserstoffes oder aliphatischen Aethers, oder eines Gemisches zweier oder mehrerer dieser Lösungsmittel, bei Temperaturen zwischen -100°C und 0°C, vorzugsweise -80°C und -30°C, mit anschliessender Behandlung des Reaktionsgemisches mit überschüssigem Wasser; siehe insbesondere Beispiel 9) sowie in der Europäischen Patentpublikation Nr. 403.945 (unter Verwendung von diversen Typen von sauren Katalysatoren sowie Lösungsmitteln, bei Temperaturen von -60°C bis 30°C; siehe insbesondere Seite 4, Zeile 48 bis Seite 5, Zeile 16 sowie Beispiel 1).

Die nachfolgenden Beispiele dienen zur näheren Erläuterung der Erfindung. Die Abkürzung GC steht für Gaschromatographie.

### Beispiel 1

Zu einer Lösung von 0,98 g (25,86 mMol) Lithiumaluminiumhydrid in 45 ml Tetrahydrofuran wird bei Raumtemperatur eine Lösung von 10 g (43,1 mMol) E,E-6-(2',6',6'-Trimethyl-cyclohex-1'-en-1'-yl)-4-methyl-hexa-1,4-dien-1-al in 30 ml Tetrahydrofuran zugetropft. Die resultierende Reaktion ist leicht exotherm. Das Gemisch wird ca. 18 1/4 Stunden unter Rühren bei Rückflusstemperatur erhitzt. Anschliessend kühlt man das Gemisch auf 10°C ab, versetzt es mit Eiswasser und stellt dessen pH-Wert auf 4 durch Zugabe von 2N Schwefelsäure. Die wässrige Phase wird dann mit Diäthyläther extrahiert und die organische Phase der Reihe nach mit Wasser, gesättigter Natriumbicarbonatlösung und gesättigter Natriumchloridlösung gewaschen. Man trocknet die vereinigten organischen Lösungen über wasserfreiem Magnesiumsulfat und engt sie unter vermindertem Druck ein. Schliesslich wird das resultierende Rohprodukt am Hochvakuum getrocknet. Man erhält auf diese Weise 10,07 g eines Gemisches von E- und Z-6-(2',6',6'-Trimethyl-cyclohex-1'-en-1'-yl)-4-methyl-hex-3-en-1-ol (E: 23%; Z: 59%; jeweils GC-Flächenprozent) sowie E- oder Z-6-(2',6',6'-Trimethyl-cyclohex-1'-en-1'-yl)-4-methyl-hex-4-en 1-ol (17% GC-Flächenprozent).
Massenspektrum (m/e):
E-6-(2',6',6'-Trimethyl-cyclohex-1'-en-1'-yl)-4-methyl-hex-3-en-1-ol:
236 (9,5), 138 (11,7), 137 (100), 136 (14,4), 121 (8,5), 107 (7,9), 95 (56,3), 93 (9,3), 81 (39,8), 79 (9,2), 69 (16,2), 67 (11,2), 57 (7,4), 55 (16,3), 53 (8,5), 43 (9,0), 41 (27,5), 31 (6,6);
Z-6-(2',6',6'-Trimethyl-cyclohex-1'-en-1'-yl)-4-methyl-hex-3-en-1-ol:
236 (4,4), 138 (11,0), 137 (100), 136 (12,1), 121 (6,6), 107 (8,1), 95 (60,5), 93 (9,3), 91 (7,2), 81 (43,8), 79 (9,3), 69 (17,8), 67 (13,3), 57 (10,2), 55 (19,5), 53 (8,8), 43 (10,9), 41 (33,3), 31 (7,2), 29 (6,9);
E- oder Z-(wahrscheinlich E-) 6-(2',6',6'-Trimethyl-cyclohex-1'-en-1'-yl)-4-methyl-hex-4-en-1-ol:
236 (28,7), 221 (8,2), 177 (13,8), 149 (5,9), 137 (100,0), 136 (25,6), 123 (31,6), 121 (40,2), 109 (16,0), 107 (29,8), 95 (72,9), 93 (29,3), 91 (17,9), 85 (17,0), 81 (58,9), 79 (21,5), 69 (32,1), 67 (22,9), 55 (35,4), 43 (21,8), 41 (53,2).

### Beispiel 2

Ein Gemisch von 0,8 g (2 mMol) Tricaprylmethylammoniumchlorid (Aliquat 336 von Fluka), 9,93 g (118,2 mMol) Natriumbicarbonat sowie 10,29g (59,11 mMol) Natriumdithionit in 65 ml Wasser wird vorgelegt. Zu diesem Gemisch wird bei Raumtemperatur eine Lösung von 1,53 g (6,57 mMol) 6-(2',6',6'-Trimethyl-cyclohex-1'-en-1'-yl)-4-methyl-hexa-2,4-dien-1-al in 65 ml Toluol innert 15 Minuten zugetropft. Man erhitzt das Reaktionsgemisch auf Rückflusstemperatur und gibt nach 22 Stunden weitere 2,29 g (13 mMol) Natriumdithionit sowie 2,21 g Natriumcarbonat zu. Nach weiteren 46,5 Stunden Erhitzen bei Rückflusstemperatur wird auf Raumtemperatur abgekühlt, mit Diäthyläther extrahiert, und die organische Phase der Reihe nach mit 0,5N Salzsäure, gesättigter Natriumbicarbonatlösung und gesättigter Natriumchloridlösung gewaschen, über wasserfreiem Magnesiumsulfat getrocknet und unter vermindertem Druck eingedampft. Das Rohprodukt (2,37 g) wird im Kugelrohr destilliert. Auf diese Weise erhält man 1,5 g eines Produktes, das nach Gaschromatographie aus E- oder Z-6-(2',6',6'-Trimethyl-cyclohex-1'-en-1'-yl)-4-methyl-hex-4-en-1-ol (30% GC-Flächenprozent), Z-6-(2',6',6'-Trimethyl-cyclohex-1'-en-1'-yl)-4-methyl-hex-3-en-1-ol (15% GC-Flächenprozent), E-6-(2',6',6'-Trimethyl-cyclohex-1'-en-1'-yl)-4-methyl-hex-3-en-1-ol (32% GC-Flächenprozent) sowie einer unbekannten Verbindung (17% GC-Flächenprozent) besteht.
Man eluiert das destillierte Produkt über 200 g Kieselgel 60 (Merck; 0,04 bis 0,06 mm) unter Verwendung von 5%igem bis 30%igem Diäthyläther in n-Hexan als Eluierungsmittel. Auf diese Weise erhält man 0,21 g eines 1:1-Gemisches von 6-(2',6',6'-Trimethyl-cyclohex-1'-en-1'-yl)-4-methyl-hexanal und vermutlich 6-(2',6',6'-Trimethyl-cyclohex-1'-en-1'-yl)-4-methyl-hex-4-en-1-al (die letzte Verbindung konnte nicht rein hergestellt werden) sowie 0,87 g eines Gemisches, das nach Gaschromatographie aus E- oder Z-6-(2',6',6'-Trimethyl-cyclohex-1'-en-1'-yl)-4-methyl-hex-4-en-1-ol (38% GC-Flächenprozent), Z-6-(2',6',6'-Trimethyl-cyclohex-1'-en-1'-yl)-4-methyl-hex-3-en-1-ol (19% GC-Flächenprozent) sowie E-6-(2',6',6'-Trimethyl-cyclohex-1'-en-1'-yl)-4-methyl-hex-3-en-1-ol (41% GC-Flächenprozent) besteht.
Physikalische Angaben:
6-(2',6',6'-Trimethyl-cyclohex-1'-en-1'-yl)-4-methyl-hexanal:
H-NMR (200 MHz, CDCl₃) 0,94 ppm (d,J=6,3H), 0,98 ppm (s,6H), 1,57 ppm (s,3H), 9,78 ppm (dd,J=2,J=2,1H);
¹³C-NMR: 19,25 ppm (q), 19,58 ppm (t), 19,81 ppm (q), 26,26 ppm (t), 28,66 ppm (q), 28,83 ppm (t), 32,77 ppm (t), 33,61 ppm (d), 34,95 ppm (s), 37,19 ppm (t), 39,90 ppm (t), 41,79 ppm (t), 126,64 ppm (s), 137,42 ppm (s), 202,74 ppm (d);
Massenspektrum (m/e):
236 (11,3), 221 (23,1), 137 (7,0), 124 (12,6), 123 (100), 127 (7,0), 109 (13,2), 107 (8,4), 95 (28,5), 93 (8,4), 81 (33,3), 79 (7,3), 69 (11,6), 67 (13,4), 55 (16,8), 43 (9,2), 41 (18,3), 29 (7,5).

Diese Verbindung kann auch leicht und in guter Ausbeute durch Hydrierung der Verbindung II in Gegenwart von Palladium auf Kohle in Aethanol hergestellt werden.

### Beispiel 3

Bei Raumtemperatur werden 6,72 g (177 mMol) Natriumborhydrid in 750 ml Isopropanol vorgelegt. Hierzu wird unter Rühren eine Lösung von 150 g (0,64 Mol) 6-(2',6',6'-Trimethyl-cyclohex-1'-en-1'-yl)-4-methyl-hexa-2,4-dien-1-al in 750 ml Isopropanol zugetropft. Nach Zugabe eines Fünftels dieser Lösung ist die Innentemperatur des Reaktionsgemisches von 26°C auf 30°C gestiegen, und es wird deshalb mit einem Eis/Wasser-Bad auf 20°C abgekühlt. Nach 1 1/4 Stunden ist die Zugabe beendet. Man rührt noch 3 Stunden bei Raumtemperatur nach, giesst dann das Gemisch auf Eis und verdünnt das wässrige Gemisch mit Diäthyläther. Unter ständigem Rühren des Gemisches werden 25 ml 2N Salzsäure zugetropft. Nach 30 Minuten Rühren neutralisiert man das Gemisch mit gesättigter Natriumbicarbonatlösung, trennt die organische Phase ab und engt sie unter vermindertem Druck ein. Der Rückstand wird dann mit Diäthyläther verdünnt und die Lösung mit wasserfreiem Magnesiumsulfat getrocknet und unter vermindertem Druck eingeengt. Auf diese Weise erhält man 135 g (91% der theoretischen Ausbeute) 6-(2',6',6'-Trimethyl-cyclohex-1'-en-1'-yl)-4-methyl-hexa-2,4-dien-1-ol.
Physikalische Angaben:
6-(2',6',6'-Trimethyl-cyclohex-1'-en-1'-yl)-4-methyl-hexa-2,4-dien-1-ol:
H-NMR (200 MHz, CDCl₃) 0,96 ppm (s,3H), 1,53 ppm (s,3H), 1,8 ppm (d,J=1Hz,3H), 2,83 ppm (d,J=7Hz,2H), 4,18 ppm (d,J=7Hz,2H), 5,35 ppm (dd,J=7Hz,J=7Hz,1H), 5,7 ppm (ddd,J=7Hz,J=7Hz,J=15Hz,1H), 6,25 ppm (d,J=15Hz,1H);
C¹³-NMR (CDCl₃): 12,26 ppm (q), 19,38 ppm (t), 19,54 ppm (q), 27,43 ppm (t), 28,12 ppm (q), 32,72 ppm (t), 34,73 ppm (s), 39,61 ppm (t), 63,42 ppm (t), 124,59 ppm (d), 127,86 ppm (s), 131,25 ppm (s), 134,20 ppm (d), 135,99 ppm (s), 136,41 ppm (d);
Massenspektrum (m/e):
234 (22,0), 203 (21,9), 145 (30,2), 133 (48,3), 123 (57,8), 119 (85,7), 110 (39,8), 105 (78,0), 95 (52,3), 93 (82,8), 91 (56,1), 81 (95,0), 77 (37,5), 69 (59,2), 67 (41,1), 55 (80,9), 43 (53,4), 41 (100).

### Beispiel 4

Bei Raumtemperatur werden 2 g (8,62 mMol) 6-(2',6',6'-Trimethyl-cyclohex-1'-en-1'-yl)-4-methyl-hexa-2,4-dien-1-al in 10 ml Toluol vorgelegt. Zum Gemisch wird 2 ml einer 70%igen Lösung von Natrium-bis-(2-methoxyäthoxy)aluminiumdihydrid (7 mMol) in Toluol gegeben, und das Reaktionsgemisch wird unter Rühren auf 90°C erwärmt. Nach 8-stündigem Rühren bei 105°C wird abgekühlt, auf Wasser gegossen und mit Diäthyläther verdünnt. Die abgetrennte organische Phase wird mit 2N Salzsäure und anschliessend gesättigter Natriumbicarbonat gewaschen, über wasserfreiem Magnesiumsulfat getrocknet und unter vermindertem Druck eingeengt. Auf diese Weise erhält man 1,89 g (89% der theoretischen Ausbeute) 6-(2',6',6'-Trimethyl-cyclohex-1'-en-1'-yl)-4-methyl-hexa-2,4-dien-1-ol.

### Beispiel 5

0,98 g (25,86 mMol) Lithiumaluminiumhydrid wird in 45 ml Diäthyläther bei Raumtemperatur vorgelegt und innert 10 Minuten eine Lösung von 10 g (43,1 mMol) 6-(2',6',6'-Trimethyl-cyclohex-1'-en-1-yl)-4-methyl-hexa-2,4-dien-1-al in 40 ml Diäthyläther zugetropft. Das Reaktionsgemisch wird 22 1/4 Stunden bei Rückflusstemperatur gerührt. Dann wird auf 8°C abgekühlt, tropfenweise mit Eiswasser versetzt und mit 2N Schwefelsäure auf pH 2 gestellt. Man extrahiert die abgetrennte wässrige Phase mit Diäthyläther, wäscht die organische Phase der Reihe nach mit Wasser, gesättigter Natriumbicarbonatlösung und gesättigter Natriumchloridlösung (zur Neutralität), trocknet sie unter wasserfreiem Magnesiumsulfat und engt sie unter vermindertem Druck ein. Auf diese Weise erhält man 10,04 g (99% der theoretischen Ausbeute) 6-(2',6',6'-Trimethyl-cyclohex-1'-en-1'-yl)-4-methyl-hexa-2,4-dien-1-ol.

### Beispiel 6

Zu einer Lösung von 0,32 g (8,45 mMol) Lithiumaluminiumhydrid in 24 ml Tetrahydrofuran wird bei Raumtemperatur eine Lösung von 2 g (9 mMol) 6-(2',6',6'-Trimethyl-cyclohex-1'-en-1'-yl)-4-methyl-hexa-2,4-dien-1-ol (nach dem in Beispiel 3, 4 oder 5 beschriebenen Verfahren hergestellt) in 5 ml Tetrahydrofuran zugetropft. Man erhitzt das Reaktionsgemisch dann auf 80°C und nach 24 Stunden versetzt es vorsichtig mit Eiswasser. Das Gemisch wird auf ein Gemisch von Diäthyläther und Eiswasser gegossen, mit Schwefelsäure auf pH 4 gestellt und mit Diäthyläther extrahiert. Die abgetrennte organische Phase wird der Reihe nach mit gesättigter Natriumbicarbonatlösung und gesättiger Natriumchloridlösung gewaschen, über wasserfreiem Magnesiumsulfat getrocknet und unter vermindertem Druck eingedampft. Auf diese Weise erhält man 1,8 g eines Gemisches von E- oder Z-6-(2',6',6'-Trimethyl-cyclohex-1'-en-1'-yl)-4-methyl-hex-4-en-1-ol (20% GC-Flächenprozent), Z-6-(2',6',6'-Trimethyl-cyclohex-1'-en-1'-yl)-4-methyl-hex-3-en-1-ol (39% GC-Flächenprozent) sowie E-6-(2',6',6'-Trimethyl-cyclohex-1'-en-1'-yl)-4-methyl-hex-3-en-1-ol (33% GC-Flächenprozent).

### Beispiel 7

Zu einem Gemisch von 2,6 g (30 mMol) Natriumbicarbonat und 0,97 g (3 mMol) Tetrabutylammoniumbromid in 5 ml Wasser und 6 ml Tetrahydrofuran werden bei Raumtemperatur 2 g (9 mMol) 6-(2',6',6'-Trimethyl-cyclohex-1'-en-1'-yl)-4-methyl-hexa-2,4-dien-1-ol (nach dem in Beispiel 3, 4 oder 5 beschriebenen Verfahren hergestellt) gegeben, und das Reaktionsgemisch wird auf Rückflusstemperatur erhitzt. Dann wird innert 30 Minuten eine Lösung von 1,3 g (15 mMol) Natriumbicarbonat und 3,3 g (19 mMol) Natriumdithionit in 20 ml Wasser zugetropft. Man erhitzt das Reaktionsgemisch 21 Stunden bei Rückflusstemperatur und gibt anschliessend die gleiche Menge der obenerwähnten Lösung zu. Auch nach weiterem 26-stündigem Rühren wird eine weitere, diesmal die doppelte Menge der obenerwähnten Lösung zugegeben. Man erhitzt noch 43 Stunden bei Rückflusstemperatur, kühlt anschliessend das Gemisch ab, extrahiert es mit Diäthyläther und wäscht die abgetrennte organische Phase mit gesättigter Natriumchloridlösung, trocknet sie über wasserfreiem Magnesiumsulfat und engt sie unter vermindertem Druck ein. Auf diese Weise erhält man 1,5 g eines Gemisches von E- und Z-6-(2',6',6'-Trimethyl-cyclohex-1'-en-1'-yl)-4-methyl-hex-3-en-1-ol (E: 31%; Z: 29%; jeweils GC-Flächenprozent) sowie unreagiertem Ausgangsmaterial 6-(2',6',6'-Trimethyl-cyclohex-1'-en-1'-yl)-4-methyl-hexa-2,4-dien-1-ol (31% GC-Flächenprozent).

### Beispiel 8

Zu 25 ml auf -69°C abgekühltem 2-Nitropropan werden innert 35 Minuten 50 g (429 mMol) Chlorsulfonsäure unter Rühren zugetropft. Dann wird innert 40 Minuten eine auf -65°C vorgekühlte Lösung von 10 g (42,37 mMol) des gemäss Beispiel 1 hergestellten Gemisches von E- und Z-6-(2',6',6'-Trimethyl-cyclohex-1'-en-1'-yl)-4-methyl-hex-3-en-1-ol (E: 23%; Z: 59%) und E- oder Z-6-(2',6',6'-Trimethyl-cyclohex-1'-en-1'-yl)-4-methyl-hex-4-en-1-ol (17%) in 100 ml Methylenchlorid zugetropft. Das Reaktionsgemisch wird 1 1/4 Stunden bei -71°C nachgerührt, dann mit 120 ml Triäthylamin unter Kühlung neutralisiert, wobei die tropfenweise Zugabe 2 Stunden dauert. Man nutscht das resultierende ausgefallene Salz ab und wäscht es mit n-Hexan. Die vereinigten organischen Phasen (u.a. n-Hexan-Spülungen) werden mit gesättigter Natriumbicarbonatlösung gewaschen, über wasserfreiem Magnesiumsulfat getrocknet und unter vermindertem Druck eingeengt. Man destilliert im Kugelrohr den Rückstand (6,35 g) und erhalt auf diese Weise 5,25 g (52,5% der theoretischen Ausbeute) eines Gemisches von (±)-[3aα,5aβ,9aα,9bβ]-Dodecahydro-3a,6,6,9a-tetramethylnaphtho[2,1-b]furan [(±)-AMBROX ®; gemäss Gaschromatographie 30% Flächenprozent] und (±)-[3aα,5aβ,9aα,9bα]-Dodecahydro-3a,6,6,9a-tetramethylnaphtho[2,1-b]furan [(±)-Epiambrox; gemäss Gaschromatographie 61% Flächenprozent].

## Patentansprüche

1. Verfahren zur Herstellung eines Gemisches von 6-(2',6',6'-Trimethyl-cyclohex-1'-en-1'-yl)-4-methyl-hex-3-en-1-ol und -4-en-1-ol, d.h. der Verbindungen der Formel 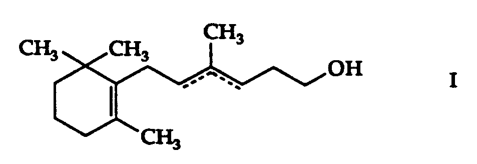 worin jeweils eine der Bindungen 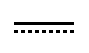 eine einfache Bindung und die andere eine Doppelbindung bezeichnet,
bzw. eines Gemisches von [3aα,5aβ,9aα,9bβ]-Dodecahydro-3a,6,6,9a-tetramethylnaphtho [2,1-b]furan und [3aα,5aβ,9aα,9bα]-Dodecahydro-3a,6,6,9a-tetramethylnaphtho[2,1-b]furan,
dadurch gekennzeichnet, dass man E,E-6-(2',6',6'-Trimethyl-cyclohex-1'-en-1'-yl)-4-methyl-hexa-2,4-dien-1-al entweder mit (a) Lithiumaluminiumhydrid oder mit (b) Natriumdithionit in Gegenwart von Natriumbicarbonat in wässriger Lösung sowie eines Phasentransferkatalysators oder mit (c) Natriumborhydrid oder mit (d) Natrium-bis (2-methoxyäthoxy)aluminiumdihydrid reduziert, und im Falle der Verwendung des Reduktionsmittels (c) oder (d) und gegebenenfalls im Falle der Verwendung des Reduktionsmittels (a) das als Zwischenprodukt entstandene E,E-6-(2',6',6'-Trimethyl-cyclohex-1'-en-1'-yl)-4-methyl-hexa-2,4-dien-1-ol, d.h. die Verbindung der Formel 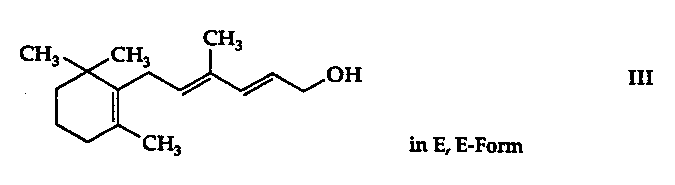 , unter Verwendung eines Reduktionsmittels (a) oder (b) zum gewünschten 6-(2',6',6'-Trimethyl-cyclohex-1'-en-1'-yl)-4-methyl-hex-3-en-1-ol und -4-en-1-ol weiter reduziert,
und gewünschtenfalls die so erhaltenen Verbindungen der Formel I in bekannter Weise in ein Gemisch von [3aα,5aβ,9aα,9bβ]-Dodecahydro-3a,6,6,9a-tetramethylnaphtho[2,1-b]furan und [3aα,5aβ,9aα,9bα]-Dodecahydro-3a,6,6,9a-tetramethylnaphtho[2,1-b]furan überführt.

2. Verfahren nach Anspruch 1, dadurch gekennzeichnet, dass man E,E-6-(2',6',6'-Trimethyl-cyclohex-1'-en-1'-yl)-4-methyl-hexa-2,4-dien-1-al mit Lithiumaluminiumhydrid in einem ätherischen Lösungsmittel bei Temperaturen zwischen 65°C und 165°C erhitzt, wobei die Verbindungen der Formel I erhalten werden.

3. Verfahren nach Anspruch 1, dadurch gekennzeichnet, dass man E,E-6-(2',6',6'-Trimethyl-cyclohex-1'-en-1'-yl)-4-methyl-hexa-2,4-dien-1-al mit Lithiumaluminiumhydrid in einem aliphatischen Aether bei Temperaturen von Raumtemperatur bis 40°C hält bzw. erwärmt, wobei die Verbindung der Formel III erhalten wird.

4. Verfahren nach Anspruch 1, dadurch gekennzeichnet, dass man E,E-6-(2',6',6'-Trimethyl-cyclohex-1'-en-1'-yl)-4-methyl-hexa-2,4-dien-1-al mit Natriumdithionit in Gegenwart von Natriumbicarbonat in wässriger Lösung, eines aliphatischen Aethers, eines aromatischen Kohlenwasserstoffes oder eines niedrigen aliphatischen Esters als zusätzlichem Lösungsmittel, sowie eines quaternären Ammoniumsalzes oder eines Phosphoniumsalzes als Phasentransferkatalysator bei der Rückflusstemperatur des Reaktionsgemisches erhitzt, wobei die Verbindungen der Formel I erhalten werden.

5. Verfahren nach Anspruch 1, dadurch gekennzeichnet, dass man E,E-6-(2',6',6'-Trimethyl-cyclohex-1'-en-1'-yl)-4-methyl-hexa-2,4-dien-1-al mit Natriumborhydrid in einem Alkohol oder wässrigem Alkohol bei der Rückflusstemperatur des Reaktionsgemisches erhitzt, wobei die Verbindung der Formel III erhalten wird.

6. Verfahren nach Anspruch 1, dadurch gekennzeichnet, dass man E,E-6-(2',6',6'-Trimethyl-cyclohex-1'-en-1'-yl)-4-methyl-hexa-2,4-dien-1-al mit Natrium-bis(2-methoxyäthoxy)aluminiumdihydrid in einem aromatischen Kohlenwasserstoff oder einem cyclischen Aether bei der Rückflusstemperatur des Reaktionsgemisches erhitzt, wobei die Verbindung der Formel III erhalten wird.

## Claims

1. A process for the manufacture of a mixture of 6-(2',6',6'-trimethyl-cyclohex-1'-en-1'-yl)-4-methyl-hex-3-en-1-ol and -4-en-1-ol, i.e. the compounds of the formula 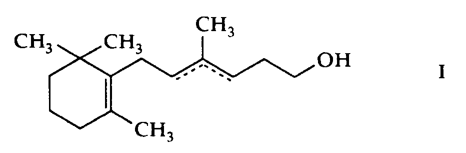 wherein one of the bonds 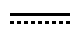 denotes a single bond and the other denotes a double bond,
or of a mixture of [3aα,5aβ,9aα,9bβ]-dodecahydro-3a,6,6,9a-tetramethylnaphtho[2,1-b]furan and [3aα,5aβ,9aα,9bα]-dodecahydro-3a,6,6,9a-tetramethylnaphtho[2,1-b]furan,
characterized by reducing E,E-6-(2',6',6'-trimethyl-cyclohex-1'-en-1'-yl)-4-methyl-hexa-2,4-dien-1-al either with (a) lithium aluminum hydride or with (b) sodium dithionite in the presence of sodium bicarbonate in aqueous solution and of a phase transfer catalyst or with (c) sodium borohydride or with (d) sodium bis(2-methoxyethoxy)aluminium dihydride and, when the reducing agent (c) or (d) is used and optionally when the reducing agent (a) is used, the E,E-6-(2',6',6'-trimethyl-cyclohex-1'-en-1'-yl)-4-methyl-hexa-2,4-dien-1-ol, i.e. the compound of the formula 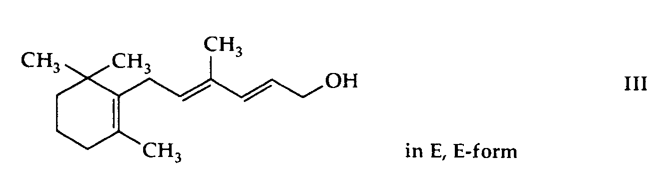 resulting as an intermediate is reduced further to the desired 6-(2',6',6'-trimethyl-cyclohex-1'-en-1'-yl)-4-methyl-hex-3-en-1-ol and -4-en-1-ol using a reducing agent (a) or (b),
and, if desired, converting the thus-obtained compound of formula I in a known manner into a mixture of [3aα,5aβ,9aα,9bβ]-dodecahydro-3a,6,6,9a-tetramethylnaphtho[2,1-b]furan and [3aα,5aβ, 9aα,9bα]-dodecahydro-3a,6,6,9a-tetramethylnaphtho[2,1-b]furan.

2. A process according to claim 1, characterized in that E,E-6-(2',6',6'-trimethyl-cyclohex-1'-en-1'-yl)-4-methyl-hexa-2,4-dien-1-al is heated with lithium aluminium hydride in an ethereal solvent at temperatures between 65°C and 165°C, whereby the compounds of formula I are obtained.

3. A process according to claim 1, characterized in that E,E-6-(2',6',6'-trimethyl-cyclohex-1'-en-1'-yl)-4-methyl-hexa-2,4-dien-1-al is held at or warmed to temperatures of room temperature to 40°C with lithium aluminium hydride in an aliphatic ether, whereby the compound of formula III is obtained.

4. A process according to claim 1, characterized in that E,E-6-(2',6',6'-trimethyl-cyclohex-1'-en-1'-yl)-4-methyl-hexa-2,4-dien-1-al is heated with sodium dithionite in the presence of sodium bicarbonate in aqueous solution, an aliphatic ether, an aromatic hydrocarbon or a lower aliphatic ester as an additional solvent and of a quaternary ammonium salt or of a phosphonium salt as the phase transfer catalyst at the reflux temperature of the reaction mixture, whereby the compounds of formula I are obtained.

5. A process according to claim 1, characterized in that E,E-6-(2',6',6'-trimethyl-cyclohex-1'-en-1'-yl)-4-methyl-hexa-2,4-dien-1-al is heated with sodium borohydride in an alcohol or aqueous alcohol at the reflux temperature of the reaction mixture, whereby the compound of formula III is obtained.

6. A process according to claim 1, characterized in that E,E-6-(2',6',6'-trimethyl-cyclohex-1'-en-1'-yl)-4-methyl-hexa-2,4-dien-1-al is heated with sodium bis(2-methoxyethoxy)aluminum dihydride in an aromatic hydrocarbon or a cyclic ether at the reflux temperature of the reaction mixture, whereby the compound of formula III is obtained.

## Revendications

1. Procédé de préparation d'un mélange de 6-(2',6',6'-triméthyl-cyclohex-1'-én-1'-yl)-4-méthyl-hex-3-én-1-ol et -4-én-1-ol, c'est-à-dire des composés de formule 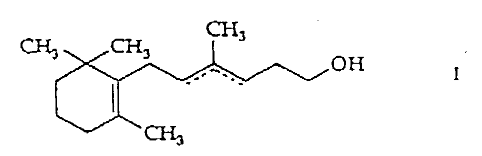 où à chaque fois une des liaisons 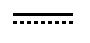 représente une liaison simple et l'autre représente une double liaison, ou selon les cas un mélange de [3aα,5aβ,9aα,9bβ]-dodécahydro-3a,6,6,9a-tétraméthylnaphto[2,1-b]furanne et de [3aα,5aβ,9aα,9bα]-dodécahydro-3a,6,6,9a-tétraméthylnaphto[2,1-b]furanne,
caractérisé en ce qu'on réduit le E,E-6-(2',6',6'-triméthyl-cyclohex-1'-én-1'-yl)-4-méthyl-hexa-2,4-diène-1-al avec (a) de l'hydrure de lithium-aluminium ou avec (b) du dithionite de sodium en présence de bicarbonate de sodium en solution aqueuse ainsi que d'un catalyseur de transfert de phase ou avec (c) du borohydrure de sodium ou avec (d) du dihydrure de sodium-bis(2-méthoxyéthoxy)aluminium, et dans le cas de l'utilisation du réducteur (c) ou (d) et le cas échéant en cas d'utilisation du réducteur (a) en ce qu'on réduit plus avant le E,E-6-(2',6',6'-triméthyl-cyclohex-1'-én-1'-yl)-4-méthyl-hexa-2,4-diène-1-ol apparu comme produit intermédiaire, c'est-à-dire le composé de formule 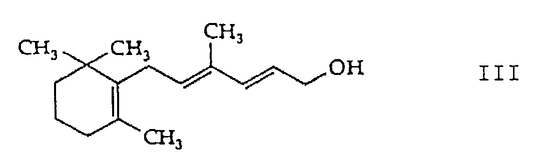 sous forme E,E en utilisant un réducteur (a) ou (b) pour donner le 6-(2',6',6'-triméthyl-cyclohex-1'-én-1'-yl)-4-méthyl-hex-3-ène-1-ol et -4-ène-1-ol,
et si on le désire en ce qu'on transforme le composé de formule I ainsi obtenu de façon connue en un mélange de [3aα,5aβ,9aα,9bβ]-dodécahydro-3a,6,6,9a-tétraméthylnaphto[2,1-b]furanne et de [3aα,5aβ,9aα,9bα]-dodécahydro-3a,6,6,9a-tétraméthylnaphto[2,1-b]furanne.

2. Procédé selon la revendication 1, caractérisé en ce qu'on chauffe le E,E-6-(2',6',6'-triméthyl-cyclohex-1'-én-1'-yl)-4-méthyl-hexa-2,4-diène-1-al avec de l'hydrure de lithium-aluminium dans un solvant éthéré à des températures comprises entre 65°C et 165°C pour obtenir le composé de formule I.

3. Procédé selon la revendication 1, caractérisé en ce qu'on maintient ou en ce qu'on chauffe le E,E-6-(2',6',6'-triméthyl-cyclohex-1'-én-1'-yl)-4-méthyl-hexa-2,4-diène-1-al avec de l'hydrure de lithium-aluminium dans un éther aliphatique à des températures comprises entre la température ambiante et 40°C pour obtenir le composé de formule III.

4. Procédé selon la revendication 1, caractérisé en ce qu'on chauffe le E,E-6-(2',6',6'-triméthyl-cyclohex-1'-én-1'-yl)-4-méthyl-hexa-2,4-diène-1-al avec du dithionite de sodium en présence de bicarbonate de sodium en solution aqueuse, dans un éther aliphatique, dans un hydrocarbure aromatique ou dans un ester aliphatique inférieur comme solvant supplémentaire, ainsi que d'un sel d'ammonium quaternaire ou d'un sel de phosphonium comme catalyseur de transfert de phase à la température de reflux du mélange réactionnel pour obtenir les composés de formule I.

5. Procédé selon la revendication 1, caractérisé en ce qu'on chauffe le E,E-6-(2',6',6'-triméthyl-cyclohex-1'-én-1'-yl)-4-méthyl-hexa-2,4-diène-1-al avec du borohydrure de sodium dans un alcool ou un alcool aqueux à la température de reflux du mélange réactionnel pour obtenir le composé de formule III.

6. Procédé selon la revendication 1, caractérisé en ce qu'on chauffe le E,E-6-(2',6',6'-triméthyl-cyclohex-1'-én-1'-yl)-4-méthyl-hexa-2,4-diène-1-al avec du dihydrure de sodium-bis(2-méthoxyéthoxy)aluminium dans un hydrocarbure aromatique ou un éther cyclique à la température de reflux du mélange réactionnel pour obtenir le composé de formule III.
